# EUROPÄISCHE PATENTANMELDUNG

(11) **EP 1 013 680 A1**
(43) Veröffentlichungstag der Anmeldung: **28.06.2000**
(21) Anmeldenummer: 99122912.1
(22) Anmeldetag: 18.11.1999
(51) Int. Cl.: C08F 246/00, C08J 7/12, C08F 299/00

(54) **Photosensitive Polymere**

(30) Priorität: 23.12.1998 DE 19859713
(71) Anmelder: Creavis Gesellschaft für Technologie und Innovation mbH, 45764 Marl (DE)
(72) Erfinder: Ottersbach, Peter Dr., 51570 Windeck (DE); Pawlik, Andreas Dr., 38159 Vechelde (DE)

(57) **Zusammenfassung**

Die Erfindung betrifft ein photosensitives Polymer, das durch Copolymerisation eines eigenschaftsbestimmenden Monomers I, eines eine photosensitive Gruppe enthaltenden Monomers II und gegebenenfalls eines weiteren Monomeren III hergestellt wird. Die erfindungsgemäßen Polymere werden zur Oberflächenbeschichtung eingesetzt und können zur Herstellung von medizinischen oder Hygieneartikeln verwendet werden.

## Beschreibung

Die Erfindung betrifft photosensitive Polymere, ein Verfahren zur deren Herstellung sowie deren Verwendung.
Die Modifizierung von Kunststoffoberflächen, speziell von technisch genutzen Produkten, ist von großem wirtschaftlichen Interesse. Als technisch und wirtschaftlich bedeutend hat sich in diesem Zusammenhang z. B. die Pfropfpolymerisation aliphatisch ungesättigter Monomere auf bereits im Markt etablierten Standardkunststoffe erwiesen, da hierdurch vollkommen neue Verwendungsmöglichkeiten gefunden werden können. Durch die Veränderungen der Oberfläche kommt man auf eine effiziente und ökonomische Art zu Produkten, mit auf den speziellen Einsatzzweck hin optimierten Grenzflächeneigenschaften. Diese veränderten Eigenschaften können hin optimierten Grenzflächeneigenschaften. Diese veränderten Eigenschaften können unter anderem zu hydrophilierten, schmutzabweisenden, bedruckbaren, lösemittelbeständigeren und flammhemmenden Oberflächen führen.
Die photochemische Pfropfpolymerisation ist in diesem Zusammenhang eine bevorzugt eingesetzte Technik.
Einen Überblick über die vielfältigen Möglichkeiten zur Eigenschaftsveränderung synthetischer Polymere durch photoinduzierte Pfropfungen liefert Jr. J. C. Arthur in Dev. Polymer Photochem. 2 (1981) 39.

So ist durch US 4 189 364 bekannt, daß sich Polymeroberflächen durch Eintauchen in eine Lösung von 2-Hydroxyethylmethacrylat und Dimethacrylat und Bestrahlung mit einer ⁶⁰Co-Quelle derartig modifizieren lassen, daß die erzeugte Oberfläche eine deutlich verbesserte Wasseraufnahme zeigt. Nachteilig an dieser Methode ist, daß der Einsatz von ⁶⁰Co-Quellen aufwendig und entsprechend kostspielig ist. Weiterhin ist die Art der von dieser Quelle ausgehenden Strahlung sehr unspezifisch und nicht auf die Oberfläche des zu modifizierenden Substrates beschränkt, was zu einer nachteiligen Beeinflussung der entsprechenden Bulkeigenschaften führen kann.

Die Aktivierung einer Oberfläche mit ultravioletter Strahlung einer Wellenlänge kleiner als 180 nm setzt neben einer entsprechend aufwendigen Bestrahlungseinheit die weitgehende Abwesenheit von Sauerstoff während der Aktivierungsphase voraus, da dieser bei der genannten Wellenlänge selber eine sehr starke Absorption zeigt. Da andererseits die Aktivierung durch diese Methode, die letztendlich auf Bildung oxidierter Reaktionsstellen beruht, einen Mindestpartialdruck an Sauerstoff voraussetzt, ist ein reproduzierbarer Aktivierungsschritt im Rahmen eines technischen Verfahrens nicht einfach realisierbar. In diesem Zusammenhang bereitet auch die kontinuierliche Intensitätsabnahme entsprechender Bestrahlungsröhren große Probleme. Daneben ist eine Veränderung der Bulkeigenschaften des Substrates durch die Bestrahlung nicht auszuschließen, da bei derartig energiereicher Strahlung auch Kohlenstoff-Kohlenstoff-Bindungen gebrochen werden können.

Eine Plasmavorbehandlung, wie sie z. B. in EP 0 574 352 beschrieben ist, stellt ebenfalls einen Vakuumprozeß dar, der im allgemeinen das Verfahren auf einen Batch-Prozeß reduziert, d. h. eine kontinuierliche Prozeßführung stark erschwert. Weiterhin ist auch hier eine entsprechend aufwendige Geräteausstattung erforderlich. Die Reproduzierbarkeit der Aktivierung ist aufgrund der Vielzahl an unabhängigen Plasmaparametern (Einsatz von Inert- oder Reaktivgasen, Energieeintrag, Behandlungsdauer) schwierig.

Die Ozonisierung einer Polymeroberfläche zur Bildung oxidierter Reaktionszentren, wie sie z. B. in US 4 311 573, US 4 589 964 oder EP 0 378 511 beschrieben ist, ist aufgrund der toxikologischen Bedenklichkeit und der Flüchtigkeit von Ozon nur unter Anwendung besondere Schutzmaßnahmen durchzuführen. Daneben besteht die Schwierigkeit der reproduzierbaren Einstellung entsprechender Ozonkonzentrationen, um im Rahmen der Qualitätssicherung eines technologischen Prozesses die gleichbleibende Qualität der hergestellten Produkte sicherstellen zu können.

Elektrische Entladungen, wie sie z. B. im Rahmen einer Korona-Behandlung zur Oberflächenaktivierung eingesetzt werden, sind aufgrund der methodenspezifischen Anforderungen im allgemeinen nur auf großflächige Substrate mit einfacher Geometrie, wie z. B. Folienbahnen oder extrudierte Profile anwendbar. Analoges läßt sich für eine Flammenbehandlung von Polymeren sagen, wobei in diesem Fall noch die stärkere thermische Belastung des Substrates hinzukommt. Einen Vergleich beider Methoden mit denkbaren und bereits realisierten Anwendungen liefert z. B. K. W.Gerstenberg in Coating 9 (1994) 324 und Coating 10 (1994) 355.

Weiterhin ist der Einsatz von Makroinitiatoren zur Oberflächenbehandlung denkbar. Die Wirkung von Makroinitiatoren beruht darauf, Polymere mit reaktiven Gruppen zu erzeugen und in einem Folgeschritt auf das zu modifizierende Substrat aufzubringen. Die Anbindung an das Substrat ist zunächst rein physikalisch. Der eigentliche Pfropfungsschritt basiert dann auf einer thermischen oder photochemischen Anregung der entsprechenden reaktiven Gruppen des Makroinitiators. Diese Methode setzt einerseits die Synthese im allgemeinen nicht kommerzieller Makroinitiatoren voraus. Andererseits muß die dauerhafte physikalische Anbindung des Makroinitiators an das jeweilige Substrat, auch unter gegebenem Lösemittel- und Temperatureinfluß, gewährleistet sein.

Ein anderes Verfahren zur Pfropfung beruht auf der Verwendung von Photinitiatoren und basiert im wesentlichen auf einer Kettenübertragung. Dieses Verfahren ist nahezu universell anwendbar. Polymerradikale oder Initiatorradikale abstrahieren dabei vom jeweiligen Substrat Wasserstoff- oder Chloratome und bilden Makroradikale, die die Pfropfpolymerisation der zugesetzten Monomeren auslösen. Wie von H. G.Elias, in Makromoleküle Bd. 1 (1990) 572 ff. beschrieben, ist die erreichbare Pfropfausbeute dabei jedoch durch die geringen Übertragungskonstanten von Polymerradikalen sehr niedrig.

Allen beschriebenen Verfahren ist gemein, daß der Oberflächenmodifizierung eine aufwendige Aktivierung mit oder ohne anschließende Pfropfpolymerisation von letztlich eigenschaftsbestimmenden Monomeren zugrunde liegt. Wünschenswert ware demgegenüber aber ein Verfahren, daß in der Lage ist, eigenschaftsbestimmende Beschichtungen, z. B. durch funktionalisierte Makromoleküle, unmittelbar und ohne Aktivierung auf die Oberfläche zu fixieren.
Der Erfindung lag daher die Aufgabe zugrunde, ein Makromolekül, das photosensitive Gruppen enthält und mit dem eine technisch und ökonomische Modifizierung von Polymeroberflächen möglich ist, zu entwickeln.

Diese Aufgabe wurde erfindungsgemäß durch die Synthese von Polymeren mit photosensitiven Gruppierungen gelöst, die zum Zwecke der Oberflächenmodifizierung auf Kunststoffoberflächen aufgebracht und durch ihre photosensitiven Gruppen durch Bestrahlung fixiert werden können.

Gegenstand der vorliegenden Erfindung sind daher photosensitive Polymere, die durch Copolymerisation eines Monomeren I mit einem eine photosensitive Gruppe enthaltenden Monomeren II erhalten werden.

Gegenstand der Erfindung ist weiterhin die Verwendung der erfindungsgemäß hergestellten photosensitiven Polymere zur Herstellung von modifizierten Oberflächen.

Das erfindungsgemäße Verfahren weist eine bemerkenswerte Kombination von Vorteilen auf. Man erzielt mit den erfindungsgemäßen photosensitiven Polymeren auf chemisch sehr verschiedenartigen Substraten dichte und gleichmäßige Beschichtungen von hervorragender Beständigkeit gegenüber Umgebungseinflüssen einschließlich von Lösemitteln und Abriebkräften. Die mit dem erfindungsgemäßen photosensitiven Polymeren bzw. Verfahren zu modifizierenden Kunststoffoberflächen müssen keine bestimmte Topographie aufweisen; Objekte mit einer dreidimensional geprägten Struktur eignen sich ebensogut wie glatte Flächen. Dies ist insbesondere bei der nachträglichen Modifizierung von vorgefertigten Gegenständen von Vorteil.

Die Herstellung der erfindungsgemäßen Polymeren erfolgt durch Copolymerisation von mindestens zwei Monomer-Komponenten.

Bei den Monomeren I handelt es sich um die eigenschaftsvermittelnde Komponente des erfindungsgemäßen Polymers. Monomer I kann ein hydrophiles oder hydrophobes Monomer sein. Hierbei kann es sich im Fall einer hydrophilen Ausstattung z.B. um Acrylsäure, Methacrylsäure, Hydroxyethylmethacrylat, Vinylpyrrolidon, Natriumstyrolsulfonat etc. handeln. Hydrophobe Eigenschaften können dem erfindungsgemäßen Polymeren zum Beispiel durch fluorhaltige Monomere I wie Tetrafluorethylen, Vinylidenfluorid, Vinylfluorid oder durch langkettige Olefine wie Octene oder Dodecene vermittelt werden.

Bei dem Monomeren II handelt es sich um die photosensitive Komponente des erfindungsgemäßen Polymers. Hierbei können Vinylketone, wie z. B. Vinylmethylketon, Methyl-Isoprenylketon, 1-Hexen-3-on, 5-Hexen-2-on, 1 Penten-3-on etc. verwendet werden.

Darüber hinaus kann die Copolymerisation mit einem zusätzlichen Monomeren III durchgeführt werden, wenn es für die angestrebte Eigenschaft des Produktes Bedeutung besitzt.

So kann, z. B. um eine bessere Verträglichkeit bei medizinischen Anwendungen zu gewährleisten, die Menge des eigenschaftsvermittelnden Monomers durch ein Comonomer wie Ethylenoxid oder Styrol vermindert werden. Weiterhin können dem erfindungsgemäßen Polymer zusätzliche Eigenschaften vermittelt werden,
z. B. Farbstoffe oder andere funktionelle Gruppen wie Isocyanate. Insbesondere kann die bakterienabweisende Wirkung eines Carboxylatgruppen enthaltenden Polymers (Monomer I: z. B. Acrylsäure) durch Zusatz von Sulfonatgruppen tragenden Monomeren wie Natriumstyrolsulfonat verbessert werden.

In einer bestimmten Ausführungsform der vorliegenden Erfindung kann als Monomer III ein Monomer aus der Gruppe der Monomeren I wie z. B. Acrylsäure, Methacrylsäure, Hydroxymethylacrylat, Vinylpyrrolidon oder Natriumstyrolsulfonat eingesetzt werden. Monomer I und III können identisch oder verschieden sein.

Die erfindungsgemäßen Polymere werden bevorzugt durch thermische Copolymerisation hergestellt. Die Copolymerisation kann auch ionisch (kathionisch oder anionisch) oder strahlenchemisch initiiert werden, jedoch wird aus Kostengründen die thermische Reaktionsführung bevorzugt werden. Die erfindungsgemäßen Polymere weisen ein Molgewicht von 2 - 1.000.000, bevorzugt 5 - 100.000 g/mol (bestimmt mit GPC) auf.

In einer weiteren Ausführungsform der vorliegenden Erfindung wird als Monomer I ein hydrophiles Monomer, wie z. B. Acrylsäure, Methacrylsäure, Hydroxymethylmethacrylat, Vinylpyrrolidon oder Natriumvinylsulfat eingesetzt.

In einer anderen Ausführungsform der vorliegenden Erfindung wird als Monomer I ein hydrophobes Monomer, wie Vinylidenfluorid, Vinylfluorid, Tetrafluorethylen, Octen oder Dodecen eingesetzt.

Zu den polymeren Substraten, deren Oberflächen erfindungsgemäß modifiziert werden, zählen Homo- und Copolymere, beispielsweise Polyolefine, wie Polyethylen (HDPE und LDPE), Polypropylen, Polyisobutylen, Polybutadien, Polyisopren, natürliche Kautschuke und Polyethylen-co-propylen; halogenhaltige Polymere, wie Polyvinylchlorid, Polyvinylidenchlorid, Polychloropren und Polytetrafluorethylen; Polymere und Copolymere aus vinylaromatischen Monomeren, wie Polystyrol, Polyvinyltoluol, Polystyrol-co-vinyltoluol, Polystyrol-co-acrylnitril, Polystyrol-co-butadien-co-acrylnitril; Polykondensate, beispielsweise Polyester, wie Polyethylenterephtalat und Polybutylenterephtalat; Polyamide, wie Polycaprolactam, Polylaurinlactam und das Polykondensat aus Hexamethylendiamin und Adipinsaure; Polyetherblockamide, z.B. aus Laurinlactam und Polyethylenglykol mit durchschnittlich 8, 12, oder 16 Ethylenoxygruppem; weiterhin Polyurethane, Polyether, Polycarbonate, Polysulfone, Polyetherketone, Polyesteramide und -imide, Polyacrylnitrtil, Polyacrylate und -methacrylate, Polysilikone. Auch Blends aus zwei oder mehr Polymeren oder Copolymeren lassen sich nach dem erfindungsgemäßen Verfahren an der Oberfläche modifizieren, ebenso wie Kombinationen aus verschiedenen Polymeren, die durch Verkleben, Verschweißen oder Zusammenschelzen miteinander verbunden sind, einschließlich der Übergangsstellen.

Weiterhin ist ein Verfahren Gegenstand der vorliegenden Erfindung, bei dem ein photosensitives Polymer gemäß einem der Ansprüche 1 bis 8 auf die zu modifizierende Polymeroberfläche aufgebracht und mit elektromagnetischer Strahlung auf dieser pfropfpolymerisiert wird.

Die Modifizierung der Polymeroberflächen geschieht derart, daß das erfindungsgemäße Polymer auf das zu modifizierende Substrat, in Reinsubstanz oder gelöst in einem Lösemittel, aufgebracht wird. Im Anschluß wird das so vorbehandelte Substrat, entweder nach einem Zwischentrocknungsschritt oder aber auch unmittelbar, mit elektromagnetischer Strahlung belichtet, so daß das erfindungsgemäße Polymer auf das Substrat gepfropt wird.

Eine verbesserte Anbindung des erfindungsgemäßen Polymers an das zu modifizierende Substrat kann erreicht werden, indem das erfindungsgemäße Polymer in einem Monomeren oder Lösungsmittel gelöst wird, das die Substratoberfläche moderat anquillt. Hierdurch kann das Polymer oder das zusätzliche Monomer in die Substratoberfläche eindringen und bei Polymerisation eine größere Anzahl von Verknüfungsstellen aufbauen. Beispielsweise können Polyurethane mit Acrylsäure innerhalb von 10-20 Sekunden bei 30 °C in ausreichendem Maße angequollen werden.

Die Propfraktion ist vermutlich eine radikalische Reaktion und führt zu fest anhaftenden, chemisch gebundenen Beschichtungen. Besonders wiederstandsfähige Beschichtungen sind daher erreichbar, wenn die Pfropfpolymerisation mit einem Vernetzer wie z. B. 1,4-Butandiol(meth)acrylat, Ethylenglycoldimethacrylat, Polyethylenstyrol (600)-diacrylat, N, N-Methylenbisacrylamid oder Divinylbenzol durchgeführt wird.

Neben der elektromagnetischen Bestrahlung ist auch eine Plasma- oder Coronabehandlung als Propfungsinitiatorschritt denkbar, bevorzugt wird jedoch der Einfachheitshalber die elektromagnetische Bestrahlung

Bewährt haben sich elektromagnetische Strahlungen mit einer Wellenlänge von 100 - 800 nm, bevorzugt 172 - 450 nm, besonders bevorzugt 270 - 330 nm. Als Strahlenquelle können Quecksilber-Hochdrucklampen, Excimer-Strahler wie z. B. von Heraeus Noblelight oder auch entsprechende Laser, wie z. B. Argonionen-, Farbstoff- oder Excimer-Laser verwendet werden.

Photosensitive Polymere, die hydrophile Monomere beeinhalten, werden zur Herstellung von hydrophilierten, bakterienabweisenden, schmutzabweisenden oder gleitreibungsverminderten Oberflächen verwendet.

Oberflächen, die besonders hydrophil sind, vermindern durch einen Wasserfilm die Gleitreibung und sind bakterienabweisend. Hydrophobe Oberflächen sind ebenfalls bakterienabweisend, zusätzlich noch schmutzabweisend, da Wassertropfen mit dem Schmutz einfach abperlen.

Weiterhin können die erfindungsgemäßen photosensitiven Polymere zur Aufbringung einer hydrophilen Sperrschicht auf ein hydrophobes Substrat verwendet werden. So lassen sich z. B. Latexkondome mit einer hydrophilen Sperrschicht ausstatten, wodurch ein Penetrieren ölhaltiger Gleitmittel in das Material behindert wird. Der umgekehrte Fall, die Verwendung zur hydrophoben Ausrüstung hydrophiler Substrate ist ebenfalls möglich. Auf diese Weise könnten z. B. Polyurethankraftstoffleitungen benzinsicher gemacht werden.

Weitere Gegenstände der vorliegenden Erfindung sind die Verwendung der erfindungsgemäß modifizierten Polymersubstrate zur Herstellung von medizintechnischen Erzeugnissen und die so hergestellten medizintechnischen Erzeugnisse als solche. Die Erzeugnisse können erfindungsgemäß modifizierte Polymersubstrate enthalten oder daraus bestehen. Solche Erzeugnisse basieren vorzugsweise auf Polyamiden, Polyurethanen, Polyetherblockamiden, Polyesteramiden oder -imiden, PVC, Polysiloxanen, Polymethacrylat oder Polyterephtalaten, die erfindungsgemäß modifizierte Oberflächen aufweisen, vorzugsweise mit Carboxyl- und Carboxylatgruppen, Sulfonatgruppen, Hydroxylgruppen und/oder Anminogruppen enthaltenden Polymeren modifizierte Oberflächen. Medizintechnische Erzeugnisse dieser Art sind beispielsweise medizinische Schläuche, Katheter, Blutbeutel, Drainagen, Führungsdrähte, Intraokularlinsen und Kontaktlinsen.

Außerdem sind Gegenstände der vorliegenden Erfindung die Verwendung der erfindungsgemäß an der Oberfläche modifizierten Polymersubstrate zur Herstellung von Hygieneerzeugnissen und die Hygieneerzeugnisse als solche. Die obigen Ausführungen über bevorzugte Materialien für medizinische Erzeugnisse gelten entsprechend. Solche Hygieneerzeugnisse sind beispielsweise Zahnbürsten, Toilettensitze, Kämme und Verpackungsmaterialien. Unter der Bezeichnung Hygieneerzeugnisse fallen auch andere Gegenstände, mit denen viele Menschen in Berührung kommen, wie Telefonhörer, Handläufe von Treppen, Tür- und Fenstergriffe sowie Haltegurte und -griffe in öffentlichen Verkehrsmitteln.

Zur weiteren Erläuterung der vorliegenden Erfindung werden die folgenden Beispiele gegeben, die die Erfindung weiter erläutern, nicht aber ihren Umfang begrenzen sollen, wie er in den Patentansprüchen dargelegt ist.

### Herstellung von Proben der erfindungsgemäßen Polymeren:

### Beispiel 1:

Dreihalskolben mit Rückflußkühler ausheizen, evakuieren und mit Argon befüllen. Diesen Vorgang dreimal wiederholen. Blasenzähler auf den Kühler aufsetzen, um Luftsauerstoff aus dem System fernzuhalten. Magnetrührfisch sowie Lösemittelgemisch aus 160 ml Isopropanol und 80 ml Toluol im Argongegenstrom in den Dreihalskolben füllen. Rührer starten und 15 g Acrylsäure, 5 g 1-Hexen-3-on und 240 mg 1,1'-Azobis(cyclohexancarbonitril) im Argongegenstrom zugeben. Die gesamte Lösung langsam zu Sieden erhitzen, danach 22 Stunden unter Argonatmosphäre refluxieren.
Anschließend Kolben abkühlen lassen, und das Reaktionsgemisch in 2 l n-Hexan ausfällen. Den Rückstand abfiltrieren und bei 50 °C im Vakuum trocknen.
Monomer-Zusammensetzung nach ¹H-NMR:
17 Mol % 1-Hexen-3-on, 79,3 Mol % Acrylsäure, Rest Lösermittel: 3,7 Mol % Isopropanol

### Beispiel 2:

Dreihalskolben mit Rückflußkühler ausheizen, evakuieren und mit Argon befüllen. Diesen Vorgang dreimal wiederholen. Blasenzähler auf den Kühler aufsetzen, um Luftsauerstoff aus dem System fernzuhalten. Magnetrührfisch sowie Löseminelgemisch aus 70 ml Isopropanol, 35 ml Toluol , 8 g Acrylsäure und 2 g 1-Hexen-3-on (im Argongegenstrom in den Dreihalskolben füllen. Rührer starten und das Gemisch auf 75 °C aufheizen. Über Tropftrichter 0,12 g 1,1'-Azobis(cyclohexancarbonitril) in einer Lösung aus 10 ml Isopropanol und 5 ml Toluol gelöst unter Argon über einen Zeitraum von 10 Minuten zugeben (Temperatur und Homogenität der Lösung beobachten). Anschließend die gesamte Lösung zum Sieden, danach 72 Stunden unter Argonatmosphäre refluxieren.
Nach Ablauf der Reaktionszeit Kolben abkühlen lassen, und das Reaktionsgemisch in 1 l n-Hexan ausfällen. Den Rückstand abfiltrieren und bei 50°C im Vakuum trocknen.
Monomeren-Zusammensetzung nach ¹H-NMR:
9 Mol % 1-Hexen-3-on, 86 Mol % Acrylsäure, Rest Lösemittel: 5 Mol % Isopropanol

### Beispiel 3:

Einen Polypropylenschlauch auf einen vorbereiteten Draht aufspannen und zuschmelzen. Im Anschluß mit einem Stück Tesafilm am Draht befestigen. Lösung aus 1,29 g des Polymeren aus Beispiel 1, 5,94 g Isopropylalkohol und 0,9 g Toluol herstellen. Den Schlauch für 10 Sekunden bei Raumtemperatur in diese Lösung tauchen, 10 Sekunden die Lösung vom Schlauch ablaufen lassen, den Schlauch in einer Drehvorrichtung arretieren (60U/min), Drehung starten und 4 Minuten belichten. Als Belichtungseinheit findet eine Excimer-Strahlungseinheit der Fa. Heraeus (Nennieistung 1000 Watt, 308 nm) Verwendung. Der Abstand zur Strahlungsquelle beträgt 4 cm. Den Schlauch anschließend für 2 Sekunden in Isopropylalkohol tauchen und dann bei 40 °C für 14 Stunden trocknen.
Oberflächenanalyse nach ESCA:
Unbeschichteter Schlauch: 99,6 Atom % Kohlenstoff, 0,4 Atom % Sauerstoff
Beschichteter Schlauch: 71 Atom % Kohlenstoff, 29 Atom % Sauerstoff

Der beschichtete Schlauch zeigt im Vergleich zur unbeschichteten Referenz eine deutlich bessere Benetzung mit entmineralisiertem Wasser.

### Beispiel 4:

Einen Polyurethanschlauch (Pellethane®) auf vorbereiteten Draht aufspannen und zuschmelzen. Im Anschluß mit einem Stück Tesafilm am Draht befestigen. Eine Lösung aus 1 g des Polymeren aus Beispiel 2 und 9 g Acrylsäure herstellen. Den Schlauch für 5 Sekunden bei Raumtemperatur in diese Lösung tauchen, 5 Sekunden die Lösung vom Schlauch ablaufen lassen, den Schlauch in einer Drehvorrichtung arretieren (60U/min), Drehung starten und 30 Sekunden belichten. Als Belichtungseinheit findet eine Excimer-Strahlungseinheit der Fa. Heraeus (Nennleistung 1000 Watt, 308 nm) Verwendung. Der Abstand zur Strahlungsquelle beträgt 4 cm.
Nach Beendigung der Bestrahlung den Schlauch zwei Stunden in VE-Wasser von 60° C legen, im Anschluß für zwei Stunden bei 60° C trocknen.
Der beschichtete Schlauch zeigt im Vergleich zur unbeschichteten Referenz eine deutlich bessere Benetzung mit entmineralisiertem Wasser.

## Patentansprüche

1. Photosensitives Polymer, erhältlich durch Copolymerisation eines Monomeren I mit einem eine photosensitive Gruppe enthaltenden Monomeren II

2. Photosensitives Polymer nach Anspruch 1,
dadurch gekennzeichnet,
daß die Copolymerisation mit einem zusätzlichen Monomeren III durchgeführt wird.

3. Photosensitives Polymer nach Anspruch 1 oder 2,
dadurch gekennzeichnet,
daß Momomer II ein Vinylketon ist.

4. Photosensitives Polymer nach einem der Ansprüche 1 bis 3,
dadurch gekennzeichnet,
daß Monomer I ein hydrophiles Monomer ist.

5. Photosensitives Polymer nach Anspruch 4,
dadurch gekennzeichnet,
daß als Monomer I Acrylsäure, Methacrylsäure, Hydroxymethylmethacrylat, Vinylpyrrolidon oder Natriumstyrolsulfonat eingesetzt wird.

6. Photosensitives Polymer nach einem der Ansprüche 1 bis 3,
dadurch gekennzeichnet,
daß Monomer I ein hydrophobes Monomer ist.

7. Photosensitives Polymer nach Anspruch 6,
dadurch gekennzeichnet,
daß als Monomer I Vinylidenfluorid, Vinylfluorid, Tetrafluorethylen, Octen oder Dodecen eingesetzt werden.

8. Photosensitives Polymer nach einem der Ansprüche 2 bis 7,
dadurch gekennzeichnet,
daß als Monomer III ein Monomer aus der Gruppe der Monomeren I eingesetzt wird.

9. Verfahren zur Modifizierung einer Polymeroberfläche,
dadurch gekennzeichnet,
daß ein photosensitives Polymer gemäß einem der Ansprüche 1 bis 8 auf die zu modifizierende Polymeroberfläche aufgebracht und mit elektromagnetischer Strahlung auf dieser pfropfpolymerisiert wird.

10. Verfahren nach Anspruch 9,
dadurch gekennzeichnet,
daß die elektromagnetische Strahlung eine Wellenlänge von 100 bis 800 nm aufweist.

11. Verfahren nach Anspruch 9,
dadurch gekennzeichnet,
daß die elektromagnetische Strahlung eine Wellenlänge von 172 bis 450 nm besitzt.

12. Verfahren nach einem der Ansprüche 9 bis 11,
dadurch gekennzeichnet,
daß die Pfropfpolymerisation mit einem Vernetzer durchgeführt wird.

13. Verwendung der photosensitiven Polymeren gemäß einem der Ansprüche 1 bis 5 zur Herstellung hydrophilierter Oberflächen.

14. Verwendung der photosensitiven Polymeren gemäß einem der Ansprüche 1 bis 8 zur Herstellung bakterienabweisender Oberflächen.

15. Verwendung der photosensitiven Polymeren gemäß einem der Ansprüche 1 bis 5 zur Herstellung gleitreibungsverminderter Oberflächen

16. Verwendung der Polymeren gemäß einem der Ansprüche 1, 2, 3, 6, 7 oder 8 zur Herstellung von schmutzabweisenden Oberflächen.

17. Verwendung der photosensitiven Polymeren gemäß einem der Ansprüche 1 bis 5 zur Aufbringung von hydrophilen Sperrschichten auf hydrophoben Substraten.

18. Verwendung der photosensitiven Polymeren gemäß einem der Ansprüche 1, 2, 3, 6, 7 oder 8 zur Aufbringung von hydrophoben Sperrschichten auf hydrophilen Substraten.

19. Verwendung der photosensitiven Polymeren gemäß einem der Ansprüche 1 bis 8 zur Herstellung von medizintechnischer Erzeugnisse.

20. Verwendung der photosensitiven Polymeren gemäß einem der Ansprüche 1 bis 8 zur Herstellung von Hygieneartikeln.
